# EUROPEAN PATENT APPLICATION

(11) **EP 1 275 381 A1**
(43) Date of publication of application: **15.01.2003**
(21) Application number: 01921849.4
(22) Date of filing: 16.04.2001
(51) Int. Cl.: A61K 9/22, A61K 31/55, A61K 47/12, A61K 47/26, A61K 47/34

(54) **TIME-RELEASE COATED SOLID COMPOSITIONS FOR ORAL ADMINISTRATION**

(30) Priority: 17.04.2000 US 198086 P
(71) Applicant: YAMANOUCHI PHARMACEUTICAL CO. LTD., Tokyo 103-8411 (JP)
(72) Inventor: SAWADA, T., c/o Yamanouchi Pharmaceutical Co., Ltd, Yaizu-shi, Shizuoka 425-0072 (JP); SAKO, K., c/o Yamanouchi Pharmaceutical Co., Ltd, Yaizu-shi, Shizuoka 425-0072 (JP); YOSHIOKA, T., c/o Yamanouchi Pharmaceut. Co., Ltd, Yaizu-shi, Shizuoka 425-0072 (JP); WATANABE, S., c/o Yamanouchi Pharmaceut. Co., Ltd, Yaizu-shi, Shizuoka 425-0072 (JP)
(74) Representative: Bates, Philip Ian
(86) International application number: JP0103229
(87) International publication number: WO01078686

(57) **Abstract**

The present invention relates to a timed-release compression-coated said composition for oral administration, said composition being a hydrgel-forming compression-coated solid preparation comprising a core tablet comprising a drug and an outer layer comprising a hydrogel-forming polymer substance and a hydrophilic base, characterized in that (1) said core tablet comprising a drug and a freely erodible filler, (2) said core tablet is capable of approximately 40 to approximately 90% erosion; and (3) said outer layer substantially does not comprise the same drug with said drug. By releasing a drug after a specific lag time, it becomes possible to effectively deliver a drug to a specific site in the digestive tract. It is therefore useful as presented as a timed-release solid composition for oral administration of a drug that is to be effectively delivered in high concentrations to the afflicted site in the lower digestive tract, a drug that is to be effectively absorbed in the lower digestive tract, a drug that is effective for chronopharmacotherapy, etc.

## Description

### TECHNICAL FIELD

The present invention pertains to a timed-release compression-coated solid composition for oral administration, in particular, a timed-release compression-coated solid composition for oral administration,, said composition being a hydrogel-forming compression-coated solid pharmaceutical preparation comprising a core tablet comprising a drug and an outer layer comprising a hydrogel-forming polymer substance and a hydrophilic base characterized in that (1) said core tablet comprising a drug and a freely erodible filler, (2) the percentage erosion of the core tablet is approximately 40 to approximately 90%, and (3) the outer layer essentially does not contain the same drug as the above-mentioned drug.

### BACKGROUND ART

A trend has been seen in recent years toward extensive research using drug delivery systems (DDS) to find a pharmaceutical preparation form appropriate for these DDS and thereby optimize pharmacotherapy. For instance, introduction of the concept of chronopharmacotherapy has been proposed, and of the attempts at effective drug absorption targeting the site of absorption, attention is being focused on the utility of the new pharmaceutical preparation technology ideal for this concept, particularly timed-release pharmaceutical preparations.

The sustained-release pharmaceutical preparation disclosed in Japanese Patent No. 2,058,495 (corresponds to EP 210,540), wherein a swelling agent is coated on granules coated with or containing drug so that it is separate from the drug and this is then coated with water-insoluble substance and which contains enough of this swelling agent to break open the film of this water-insoluble substance once a specific amount of time has passed, is an example of a timed-release noncompression-coated pharmaceutical preparation. Although the pharmaceutical preparation of this invention showed a good timed release pattern *in vitro* and *in vivo,* there is a possibility of low bioavailability.

Moreover, an intestinal delivery oral pharmaceutical preparation, which controls release initiation time, that is, an oral pharmaceutical preparation comprising a core containing acidic substance, a first layer coating the core made from a water-insoluble polymer and which can also comprise a water-soluble substance, a second layer coating the first layer containing a main drug, a third layer coating the second layer made from a low pH-dissolving polymer, and a fourth layer coating the third layer made from an enteric polymer, is disclosed in a published Japanese Patent Application (Kokai) No. Hei 7-10,745 as another timed-release noncompression-coated pharmaceutical preparation. This invention is an attempt at timed release using a multilayered film, but conducting the coating procedure multiple times is essential to this technology. Actually, when the purpose is to obtain a multilayered coating in the strict sense of the word, that is, to obtain an intricate multilayered coating, complexity of the pharmaceutical preparation design in terms of selecting the coating base, selecting the coating solvent, selecting the coating method,-determining the middle layers, etc., and complexity of production are unavoidable when making a pharmaceutical preparation that uses the properties of each coating layer.

On the other hand, the inventors report on a hydrogel sustained-release pharmaceutical preparation made from a drug, a hydrophilic base, and a hydrogel-forming polymer substance with which good drug release is possible in the upper digestive tract as well as the colon of the lower digestive tract in International Publication No. WO 94/06414 (corresponds to EP No. 066,1045A1 and Japanese Patent No. 3,140,465.)

The inventors further performed fundamental studies of timed-release pharmaceutical preparations and studies for the purpose of applying timed-release pharmaceutical preparations to insulin during the course of proceeding with studies of hydrogel sustained-release pharmaceutical preparations and report that 2-hour timed-release was effective with insulin and, as information obtained from the fundamental studies, that addition of polyethylene glycol of high solubility in water to the core tablet is effective and that timed release can be adjusted by varying the mixture ratio of polyethylene glycol and polyethylene oxide in the outer layer (November 18, 1998, American Association of Pharmaceutical Scientists Annual Meeting: Exploitation of Novel Timed-Release Dosage Forms for Oral Delivery of Peptide Drugs).

Recently, drugs are rarely used singularly as a result of diversification of medicine and changes in patient phase with aging, and in many cases multiple drugs are administered simultaneously or at staggered administration times. There are drugs that interact in terms of pharmacokinetics with drugs that are administered concomitantly. Pharmacokinetic drug interaction almost always demonstrates because the drugs themselves compete for one route, for example enzymes, carriers, etc., when drugs that use the same routes in terms of drugs absorption, distribution, metabolism or excretion are used concomitantly.

Means for averting drug interaction with an administration protocol whereby the times of administration of concomitant drugs to a patient are staggered is disclosed in "Yakubutsu Yosokugaku Nyumon" (Yasufumi Sawada, Yakugyo Jiho Publishers, August 31, 1993). The administration protocol whereby metal cation-containing antacids (magnesium, aluminum, etc.) and new quinolone agents (norfloxacine, etc.) are administered as much as 6 or 7 times/day with the administration times strictly specified is an example. However, taking into consideration patient compliance, another, more realistic means of averting drug interaction is desirable.

### DISCLOSURE OF THE INVENTION

In light of such technological standards, the inventors studied the use of compression-coated tablets with a simple pharmaceutical preparation design and with which production of the pharmaceutical preparation is not complex focusing on the use of timed-release pharmaceutical preparations, which thus far have not been used, as a means of averting various pharmacokinetic drug interactions and as a result, they discovered that compression-coated tablets with a high percentage erosion of the core tablet show high bioavailability, even after timed release.

The inventors discovered as a result of further intense studies in order to explain the reason for the above-mentioned and develop an ideal timed-release pharmaceutical preparation that surprisingly, there are fillers suitable for use with core tablets, depending on the drug, that is, that the effect of good erosion of the core tablet without a reduction in bioavailability as a timed-release pharmaceutical preparation can be realized by adding an organic acid with excellent water solubility, such as malic acid, tartaric acid, citric acid, etc., as the filler added to the core tablet. As a result of proceeding to analyze this phenomenon further, the inventors discovered that a percentage erosion of the core tablet of approximately 40 to approximately 90% is necessary and selecting the drug, freely erodible filler, and other additives and specifying the amounts added so that this percentage erosion is obtained are essential to making the ideal timed-release pharmaceutical preparation.

The present invention was completed based on these discoveries and relates to a timed-release compression-coated solid composition for oral administration comprising a core tablet comprising a drug and an outer layer comprising a hydrogel-forming polymer substance and a hydrophilic base, said composition being a hydrogel-forming compression-coated solid pharmaceutical preparation, wherein (1) said core tablet comprising a drug and a freely erodible filler, (2) the percentage erosion of the core tablet is approximately 40 to approximately 90%, and (3) the outer layer essentially does not contain the same drug as the above-mentioned drug.

The composition of the present invention is particularly characterized in that A. it absorbs the water in the upper digestive tract so that the outer layer all but completely gels, B. the water penetrates inside the tablet and a solution state or suspension state is produced once the core tablet has been eroded, C. the gelled outer layer is eroded as it moves to the lower digestive tract, and D. further, part of the outer layer is disintegrated or peeled so that drug is released. Ideal timed release of a drug can be realized by this type of structure, even in the lower digestive tract with a low water content, regardless of the properties of the drug.

The present invention further relates to a method of reducing undesirable pharmacokinetic drug interaction between a drug and another concomitant drug that use the same route for *in vivo* drug absorption, distribution, metabolism or excretion in humans by using the above-mentioned timed-release compression-coated solid composition for oral administration.

The oral drug pharmaceutical preparation and method of reducing drug interaction of the same of the present invention will now be described in further detail:

The outer layer of the present invention can contain a drug that is not the purpose of timed release. For instance, a drug other than the drug that is the purpose of timed release released in the lower digestive tract and which can be released in the upper digestive tract can be contained in the outer layer of the compression-coated tablet.

The entry "the outer layer essentially does not contain the same drug as the above-mentioned drug" means that the outer layer can contain the same drug as the above-mentioned drug as long as it is within a range in which the results of the timed-release pharmaceutical preparation of the present invention are not lost. Furthermore, the embodiment wherein the outer layer preferably does not contain the drug that is the object of timed release is ideal in the case of a timed-release pharmaceutical preparation for averting drug interaction.

"Upper digestive tract" in the present invention means the part from the stomach to the duodenum and jejunum "lower digestive tract" means the part from the ileum to the colon.

A drug with which conversion to a timed-release pharmaceutical preparation is effective in order to accomplish the purpose of the present invention, such as drugs that should not be simultaneously administered because they have pharmacokinetic drug interaction, particularly one of drugs that induce and/or inhibit drug metabolism by cytochrome P450, particularly CYP3A4, in the small intestine, such as duodenum, jejunum, etc., and/or liver, beginning with drugs that are brought to be effectively transported in high concentrations to the afflicted site of the lower digestive tract, drugs that are brought to be effectively absorbed in the lower digestive tract, and drugs that are effective for chronopharmacotherapy, are selected as the drug used in the present invention.

Salazosulfapyridine, 5-aminosalicylic acid, cortisone acetate, triamcinolone, dexamethasone, budesonide, tegafur, fluorouracil, and their derivatives, etc., which are drugs for the treatment of Crohn's disease, ulcerative colitis, hypersensitivity colitis, colon cancer, etc., are examples of drugs that it is effective to deliver it to the afflicted site of the lower digestive tract in high concentrations.

Drugs that are sparingly absorbed orally because of interaction with the digestive tract mucous membrane and/or mucous layer, etc., are examples of drugs that the absorption at the lower digestive tract is effective. It can be a drug that is hardly absorbed from the digestive tract because of poor permeability of the mucous layer present on the digestive tract mucous membrane, a drug that is difficult to absorb due to interaction with a substance present in the mucous layer, a drug that is difficult to absorb from the digestive tract because of poor permeability in the digestive tract mucous membrane, etc. For instance, extracts derived from flora and fauna present in nature (such as extract, tincture, etc.), or compounds isolated from extract or chemically synthesized compounds, etc., and the like are included. The drug can be a single compound, or it can be a mixture of two or more types. Moreover, if the drug is a compound, salts of the compound, various solvents of said compound that are pharmaceutically acceptable (for instance, water, etc.), and solvents of salts of said compound are included. Moreover, crystal polymorphs of the same are also included. Further, if there is an asymmetric carbon present in the structure of the compound and there are optical isomers or stereoisomers based on it, these optical isomers, stereoisomers, and mixtures of these isomers are all included. There are no particular restrictions to the salts of the compound as long as they are pharmaceutically acceptable. Specific examples are mineral acid salts, such as hydrochlorides, hydrobromides, hydroiodides, phosphates, nitrates, sulfates, etc., organic sulfonates, such as methanesulfonates, ethanesulfonates, 2-hydroxyethanesulfonates, p-toluenesulfonates, etc., organic carboxylates, such as acetates, propionates, oxalates, malonates, succinates, glutarates, adipates, tartrates, maleates, malates, mandelates, etc., and the like.

Specifically, the drug used can be an osteoporosis drug, a bone metabolism-improving agent, a hypnotic sedative, a sleep-inducing agent, an anti-anxiety agent, an anti-epilepsy agent, an antidepressant, an anti-Parkinson's agent, an agent used for the treatment of psychoneurosis, an agent used for the treatment of central nervous system disorders, a local anesthetic, a skeletal muscle relaxant, an agent used in the treatment of autonomic nervous system disorders, an anti-inflammatory antipyretic analgesic, a spasmolytic, an anti-vertigo agent, a cardiotonic, an agent for treatment of arrhythmia, a diuretic, a hypotensive, a vasoconstrictor, a vasodilator, a drug for treatment of circulatory disorders, an agent for hyperlipidemia, an agent that promotes respiration, an antitussive, an expectorant, an antitussive expectorant, a bronchodilator, an antidiarrheal agent, an agent for controlling intestinal function, an agent for treatment of peptic ulcers, an antacid, a laxative, a cholagogue, a gastrointestinal drug, an adrenocortical hormone, a hormone, an agent for treatment of urogenital disorders, a vitamin, a hemostatic, an agent for treating liver disease, an agent for treatment of gout, an agent for treatment of diabetes, an antihistamine, an antibiotic, an antibacterial, an anti-malignant tumor agent, a chemotherapeutic agent, a multisymptom cold agent, a nutrition-enhancing health agent, etc. Examples are bisphosphonate compounds (incadronate, [(cycloheptylamino)-methylene]bis-phosphonate), YM175; produced by the method in examined Japanese Patent Publication (Kokoku) No. Hei 7-629), minodronic acid, [1-hydroxy-2-imidazo(1,2-a)pyridin-3-ylethylidene] bisphosphonate), YM529; produced by the method entered in examined Japanese Patent Publication (Kokoku) No. Hei 6-99457), alendronate, ibandronate, etidronate, olpadronate, chlodronate, zoledronate, tiludronate, neridronate, pamidronate, risedronate, [1-hydroxy-3-(1-pyrrolidinyl)-propylidene]bis-phosphonate, etc.), 5-aminosalicylic acid, acyclovir, adinazolam, ascrobic acid, aspirin, acetylsalicylic acid, acetaminophen, acetobutol, acetohexamide, atenolol, atorvastatin, apomorphine, aminopyrine, aminophylline, ethyl aminobenzoate, amrinone, amobarbital, albuterol, alprazolam, allopurinol, ampicillin, ambroxole isoniazide, idebenone, ibuprofen, indeloxazine, indomethacin, ethenzamide, ethosuccimide, etomidoline, enalapril, ephedrine, erythromycin, oxytetracycline, oxyphenbutazone, osalazine, omeprazole, carmofur, quinidine, glibizide, chloramphenicol, chlordiazepoxide, chlorthiazide, ketoconazole, codeine, cobamamide, colchicine, zafirlukast, diazepam, digitoxin, diclofenac, diclofenac sodium, cyclophosphamide, digoxin, cycotiamine, dipyridamole, cimetidine, josamycine, simvastatin, sucralfate, spironolactone, sulpiride, sulfasalazine, sulfmethoxazole, sulfisoxazole, cefotetan, cefuroxime, selegiline, celecoxib, tasosartan, thiotepa, theophylline, dextromethorphan, tetracycline, tepronone, terfenadine, terbutaline, doxorubicin, tramadole, etodolac, triamcinolone, triamterene, torbutamide, nadolol, naproxen, nicotinamide, nitroglycerin, nitrofurantoin, nifedipine, nemonapride, noscapine, hydrocortisone, vardecoxib, sodium valproate, haloperidol, hydrochlorothiazide, hydrocortisone, pilocarpine, famotidine, phemacetin, phenytoin, phenylbutazone, phenyl propanolamine, phenobarbital, fenoprofen calcium, pseudoephedrine, budesonide, formoterol fumarate, praunotol, pravastatin, pravastatin sodium, pranrucast, purimidone, fluorouracil, prednisolone, prednisone, procainamide, prostaglandin I derivatives, such as beraprost sodium, etc., furosemide, probenecid, bromvaleryl urea, betamethasone, penicillin, peroxetin, perfphenazine, benzyl penicillin, pentazocine, calcium homopanthothenate, polythiazide, chlorophenylamine maleate, midazolam, milnacidran, doxazocin mesilyate, methyl dopa, methylphenidate, methoclopramide, methotrexate, methoprolol, mepiprizole, morphine, ranitidine, lansoprazole, lisinopril, risperidone, griseofulvin, lidocaine, codeine phosphate, dimemorfan phosphate, pyridoxal phosphate, reserpine, levo dopa, lovastatin, lorazepam, warfarin, aclarubicin hydrochloride, azasetron hydrochloride, amitriptyline hydrochloride, amosulalol hydrochloride, talampicillin hydrochloride, indenolol hydrochloride, ethambutol hydrochloride, ondansetron hydrochloride, granisetron hydrochloride, chloropromazine hydrochloride, diphenhydramine hydrochloride, dibucaine hydrochloride, tamsulasin hydrochloride, thiapride hydrochloride, terazosine hydrochlorice, nicardipine hydrochloride, barnidipine hydrochloride, hydralazine hydrochloride, bifemerane hydrochloride, prazosin hydrochloride, propafenone hydrochloride, moperone hydrochloride, ranitidine hydrochloride, ramosetron hydrochloride, butyl scopolamine bromide, isosorbid nitrate, quinidine nitrate, guanetidine nitrate, thiamine nitrate, tocopherol acetate, chloral hydrate, N-[4-[(1-acetimideyl-4-piperidyl)oxy]phenyl]-N-[(7-amidino-2-naphthyl)methyl] sulfamoyl acetate monomethyl sulfonate (produced by the method entered in International Publication No. WO 96/16940; compound that inhibits active blood coagulation factor X and useful as a blood coagulation-inhibiting agent and preventive and therapeutic agent for blood clots), etc. Other examples are peptides, proteins, and their derivatives that are freely decomposed in the upper digestive tract, such as insulin, calcitonin, angiotensin, vasopressin, desmopressin, LH-RH (leutinizing hormone releasing hormone), somatostatin, glucagon, oxytocin, gastrin, cyclosporin, somatomedin, secretin, h-ANP (human atrial natriuretic peptide), ACTH (adrenocorticotropic hormone), MSH (melanophore-stimulating hormone), β-endorphin, muramyl dipeptide, enkephalin, neurotensin, bombesin, VIP (vasoactive intestinal peptide), CCK-8 (cholecystokinin-8), PTH (parathyroid hormone), CGRP (calcitonin gene-related peptide), TRH (thyrotropin-releasing hormone), endoserin, hGH (human growth hormone), cytokines, such as interluekin, interferon, colony stimulating factor, tumor necrosis factor etc.

Moreover, drugs that are effective therapeutically for obtaining the blood concentration needed for a necessary time period when biorhythm of the antipyretic analgesics of acetaminophen, indomethacine, hydrocortisone, ibuprofen, salazoprin, etc., is known, such as antitussive expectorants including theophylline, etc., vasodilators, including nicardipine hydrochloride and nifedipine, etc., and coronary vasodilators, including isosorbide nitrate, etc., can be given as drugs that are effective for chronopharmacotherapy. Drugs that should be at a particular blood concentration at a specific time period at night or early in the morning are ideal.

Drugs that have pharmacokinetic drug interaction are further classified based on the type of interaction. In general, there are no particular restrictions as long as they are drugs that use the same route as other concomitant drugs or food for absorption, distribution, metabolism or excretion and they display undesirable pharmacokinetic interaction.

Each of the above-mentioned drugs can be used in free form or as a salt that is pharmaceutically acceptable. Moreover, when necessary, 2 or more drugs can be used in combination with one another.

Drugs that display pharmacokinetic drug interaction in particular are described in further detail below:

### (a) Interaction in terms of drug metabolism

Drugs are deactivated or converted to water-soluble metabolites that are readily excreted via the kidneys by the effects of drug-metabolizing enzymes in the liver, etc. Cytochrome P450 (CYP) is said to be the most important drug-metabolizing enzyme. It is said that approximately 70% of pharmacokinetic drug interaction is around drug metabolism, and of this, 95% or more is interaction via CYP. Many molecular species of CYP exist, and those that play the most important role in drug metabolism are CYP1A2, CYP2C9, CYP2C19, CYP2D6 and CYP3A4. The molecular species of CYP involved in drug metabolism is determined by the chemical structure of the drug. Moreover, the molecular species of CYP involved in metabolism varies with each site in the chemical structure, and there are also drugs that are metabolized by multiple molecular species of CYP.

Theophylline, caffeine, phenacetin, clomipramine, imipramine, fluvoxamine, zolpidem, clozapine, proprapanolol, propafenone, chlorzoxazone, tacrine, acetaminophen, ondansterone, verapamil, etc., are drugs that are metabolized by CYP1A2 and/or drugs that inhibit CYP1A2.

Diclofenac, naproxen, ibuprofen, piroxicam, flurbiprofen, indomethacin, phenytoin, carbamazepin, tolbutamide, glibenclamide, glipizide, glimepiride, warfarin, losartan, torsemide, dronabinol, tenoxicam, mefanamic acid, sulfafenazole, etc., are drugs that are metabolized by CYP2C9 and/or drugs that inhibit CYP2C9.

Mephenytoin, diazepam, phenytoin, phenobarbital, hexobarbital, mephobarbital, omeprazole, lansoprazole, proguanil, amitriptyline, clomipramine, imipramine, sitalopram, propranolol, thiridazine, carisoprodol, warfarin, nirvanol, etc., are drugs metabolized by CYP2C19 and/or drugs that inhibit CYP2C19.

Propafenone, flekainid, mexiletine, enkainid, spartein, N-propylajmaline, ajmaline, metoprolol, timolol, pindolol, propranolol, bufuralol, perbutolol, popindolol, alprenolol, carbedilol, debrisokin, indolamine, guanoxan, urapidil, nicergoline, risperidone, thioridazine, perphenazine, clozapine, trifluperiol, fluphenazine, chlorpromazine, haloperidol, clomipramine, nortriptyline, amitriptyline, imipramine, trimipramine, desipramine, zolpidem, brofaromine, amiframine, paroxetine, fluoxetine, maprotiline, banrafaxin, fluvoxamin, trazadone, tomoxetin, dihydrocodeine, oxycodeine, codeine, tramadol, dextromethorphan, femformine, perhexelin, chlomiopran, quinidine, cimetidine, ondansteron, etc., are drugs that are metabolized by CYP2D6 and/or drugs that inhibit CYP2D6.

Anfentanyl, fentanyl, sulfentanyl, cocaine, dihydrocodeine, oxycodeine, tramadol, erythromycin, clarithromycin, troleandomycin, azithromycin, itraconazole, ketoconazole, dapsone, midazolam, triazolam, alprazolam, diazepam, zolpidem, felodipine, nifedipine, nitrendipine, amlodipine, isradipine, nicardipine, nimodipine, nisoldipine, nildipine, bepridil, diltiazem, verapamil, astemizole, terfenadine, loratidine, cyclosporine, tacrolimus, rapamycin, amiodarone, disopyramide, lidocaine, propafenone, quinidine, imipramine, amitriptyline, clomipramine, nafazodone, sertraline, trazodone, haloperidol, pimozide, carbamazepine, ethosuximide, trimethadione, simvastatin, lovastatin, fluvastatin, atrovastatin, etoposide, ifosfamide, paclitaxel, tamoxifen, taxol, vinblastine, vincristine, indinavir, ritonavir, saquinavir, testosterone, prednisolone, methylprednisolone, dexamethasone, proguanil, warfarin, finasteride, flutamide, ondansteron, zatosetron, cisapride, cortisol, zonisamide, desmethyldiazepam, conivaptan, etc., are drugs that are metabolized by CYP3A4 and/or drugs that inhibit CYP3A4 (Sogo Rinsho, 48(6), 1427-1431, 1999/ Seishinka Chiryogaku, 14(9), 951-960, 1999).

It appears that when multiple drugs that are metabolized by CYP or inhibit CYP of the same molecular species in this way compete for these metabolizing enzymes, the extent of this same competition varies with the affinity of the drug for the CYP, but metabolism will be inhibited in some way. Metabolism of drugs that have poorer affinity for the CYP will be inhibited and as a result, there will be drug interaction in the form of elevated blood concentration, prolonged blood half-life, etc.

For instance, inhibited metabolism, resulting in a rise in the blood concentration, of midazolam and terfenadine, cyclosporine, etc., by erythromycin, of methyl prednisolone by ketoconazole, and of lovastatin by itraconazole are examples of concomitant use of drugs that inhibits metabolism by CYP3A4.

Moreover, there are cases where foods that are metabolized by the same species of CYP as drugs compete for the same metabolizing enzymes to inhibit in some way the metabolism of these drugs. Moreover, there are also foods that inhibit a specific molecular species of CYP. For instance, components contained in grapefruit juice inhibit CYP3A4 and therefore, interaction resulting in elevated blood concentrations of the drugs is seen when cyclosporine and tacrolimus, midazolam, triazolam, terfenadine, etc., which are metabolized by CYP3A4, are taken with grapefruit juice.

On the other hand, it is known that there are drugs that induce drug-metabolizing enzymes. For instance, rifampicin induces CYP3A4, CYP2C9 and CYP2C19 to promote metabolism of nifedipine, warfarin, diazepam, cyclosporine, disopyramide, torbutamide, ethinyl estradiol, etc., and reduce blood concentrations.

### (b) Interaction in terms of drug absorption

The route of absorption of drugs is also by the skin or oral mucosa, etc., but the main route of absorption is by the digestive tract.

Changes in gastric pH due to the effect of other drugs used concomitantly changes solubility of drugs and controls or promotes absorption from the digestive tract. For instance, gastric pH rises to 3 to 5 with administration of cimetidine during concomitant use of cimetidine and ketoconazole and as a result, there is a reduction in solubility of ketoconazole and absorption via the digestive tract is inhibited, leading to a reduction in the blood concentration.

Moreover, there are cases where when a drug is actively absorbed with concomitant drugs via the same carriers on the epithelial cells of the small intestines, absorption of the concomitant drugs is inhibited by this drug. For instance, it is reported that there is a reduction by approximately half in the cefadroxil plasma concentration when cefadroxil, a betalactam antibiotic, is concomitantly administered with cefalexine. This reduction in the blood concentration is apparently due to inhibition as a result of competition for the carrier by the two drugs.

### (c) Interaction in terms of drug distribution

Drugs that have been absorbed via the digestive tract or have moved to the blood from the site of administration are distributed to blood cells at a specific ratio, or bind with proteins in plasma. The free fraction of the drug is distributed to each tissue to realize pharmacological action and therefore, when drug bound to protein is expelled from this binding site and interaction occurs so that the concentration of the free fraction of the drug rises, this pharmacological effect is enhanced. For instance, warfarin, torbutamide, etc., are released from the protein binding site, resulting in a rise in the concentration of the free fraction of the drug, when they are concomitantly used with aspirin, etc.

Moreover, P glycoproteins are found in the cells of the mucosa of the small intestines, cells of the uriniferous tubules, and endothelial cells of the capillaries of the brain, and they have the mechanism of transporting many drugs to outside the cells. When a drug that inhibits P glycoprotein is concomitantly used with a drug that is transported via P glycoprotein, there are cases where secretion of drugs into the intestines, transporting drugs out of the brain, and excretion in urine are inhibited. Vinblastin, vincristin, doxorubicin, etoposide, taxol, adriamycin, dexamethasone, hydrocortisone, verapamil, diltiazem, nifedipine, nicardipine, cyclosprin, tacrolimus, acebutolol, metoprolol, nadolol, timolol, prostaglandin, rodamine 123, digoxin, colchicine, dideoxyforscolin, etc., are drugs that are transported out by P glycoproteins. Etoposide, hydrocortisone, progesterone, testosterone, verapamil, diltiazem, nifedipine, felodipine, nitrendipine, nicardipine, cyclosporine, tacrolimus, amiodarone, lidocaine, quinidine, itraconazole, ketoconazole, erythromycin, tamoxifen, terfenadine, clorpromazine, selprolol, diprofloxacine, spironolactone are drugs that inhibit P glycoproteins ("Rinshou Yakubutsu Doutaigaku," Revised Version 2," Chapter II. Absorption of drugs from site of administration, page 19, Ryuichi Kato, author, Nankodo Publishers).

### (d) Interaction in terms of excretion of drugs.

Drugs that have entered the body are excreted into the urine by the kidneys and are secreted and re-absorbed in the uriniferous tubules. Anionic carriers and cationic carriers participate in secretion from the uriniferous tubules. There is a possibility that drugs that use the same carrier will interact with one another. Probenecid, diodrast, acetazolamide, etc., are drugs that inhibit secretion via anionic carriers. Quinine, methyl nicotinamide, tolazolline, tetramethyl ammonium, etc., are drugs that inhibit secretion via cationic carriers.

On the other hand, when re-absorption from the uriniferous tubules is inhibited, there is an increase in the amount excreted in urine and this lowers the blood concentration. For instance, re-absorption of chlorpropamide from the uriniferous tubules is inhibited by concomitant use with sodium bicarbonate.

Diltiazem, ketoconazole, acetaminophen, midazolam, simvastatin, and conivaptan are typical examples of drugs with which timed release control of the present invention is effective and the results of the present invention are confirmed. However, it is clear based on the technical concept that is clarified by the test examples given later that the composition of the present invention can be applied to any drug with which timed release control is effective.

Drugs metabolized by CYP3A4 are typical examples of drugs with which timed release control is effective, and in addition to the above-mentioned examples of drugs, compounds metabolized by CYP3A4, including the nitrogen-containing aromatic 5-member cyclocondensed benzazepine derivatives represented by the following general formula (I) or their pharmaceutically acceptable salts, can be mentioned.

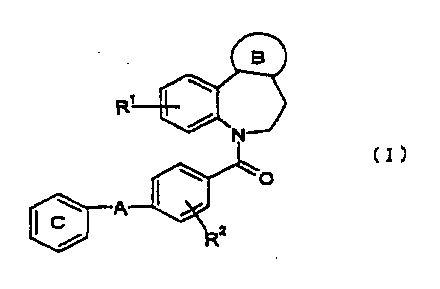

The symbols in the formula are defined below:
Ring B: a nitrogen-containing aromatic 5-member ring, which may have substituents, has at least 1 nitrogen atom, and may further have 1 oxygen or sulfur atom.
R¹, R²: which may be the same or different, are selected from hydrogen atoms, halogen atoms, lower alkyl groups, lower alkyl-O-groups, or amino groups that may be substituted with lower alkyl group(s)
A: a single bond or a group represented by the formula -(CR³R⁴)ₙ-CONH-.
n: 0 or an integer of 1 to 3.
R³, R⁴: which may be the same or different, are selected from hydrogen atoms or lower alkyl groups (provided that the R³ and R⁴ may form a lower alkylene group having 2 to 7 carbon atoms).
Ring C: a benzene ring which may have substituent(s).

The nitrogen-containing aromatic 5-member cyclocondensed benzazepine derivatives represented by above-mentioned general formula (I) and their salts include all compounds implied by the general formula in International Patent Publication No. 95/03305, and typical examples of drugs with which timed release control is effective that are used in the present invention are the 4'-[(2-methyl-1,4,5,6-tetrahydroimidazo[4,5-d][1]benzazepin-6-yl)carbonyl]-2-phenylbenzanilide entered in International Publication No. WO 94/03305 and its pharmaceutically acceptable salts.

These compounds can be easily obtained by the production method entered in above-mentioned International Publication No. WO 95/03305, or by production in accordance with this method.

Furthermore, as is clear based on the technical concept of the present invention, particularly the examples, as long as the results of the present invention are realized, the drug used in the present invention is of course not limited to only those drugs for which conversion to a timed-release pharmaceutical preparation is known to be desirable at the present time.

The mixture ratio of the drug used in the present invention should be the effective dose per unit pharmaceutical preparation of this drug that is usually used pharmacologically for treatment or prevention and cannot be generally specified. However, it is preferably approximately 75 wt% or less, particularly approximately 50 wt% or less, of the total pharmaceutical preparation.

The term percentage erosion used in the present invention is specified as necessary for guaranteeing bioavailability after timed release of the drug, and is also used in order to define the filler that is freely erosion of the present invention. That is, the percentage erosoin means the ratio of core tablet that has been eroded once the compression-coated tablet has been moistened for a specific amount of time and is determined by the method of determination of the percentage erosion described below. Moreover, a percentage erosion of approximately 40 to approximately 90%, preferably approximately 45 to 85%, particularly approximately 50 to approximately 80%, means that the filler contained in a core tablet of a compression-coated tablet showing this percentage erosion is a freely erodible filler for the drug contained in this core tablet. Moreover, a percentage erosion of approximately 40 to approximately 90% means that bioavailability after timed release of the drug is guaranteed. Specifically, if the percentage erosion is less than approximately 40%, sufficient release of the drug will not be obtained and there is a chance of a reduction in bioavailability. Moreover, if the percentage erosion is greater than approximately 90%, there will be a reduction in strength of the entire pharmaceutical preparation and therefore, there is a chance that the drug will be released sooner than expected in the upper digestive tract and the purpose of administration cannot be accomplished. The percentage erosion is determined by the following method:

### Determination of percentage erosion

A compression-coated tablet containing drug is made in accordance with the method of producing a timed-release compression-coated solid composition disclosed in the present invention, the tablet is moistened for 3 hours in water at 37°C, then the gelled part of the tablet is peeled off and the portion of the core tablet that has not eroded is removed. The core tablet is allowed to dry overnight in a dryer at 40°C and weight is determined. The value obtained by subtracting dry weight from initial core tablet weight and dividing this by initial core weight is multiplied by 100 to calculate the percentage erosion.

There are no particular restrictions to the "freely erodible filler" used in the core tablet of the present invention as long as it is usually allowed pharmaceutically and shows the percentage erosion specified by the present invention when combined with the drugs and other fillers that will be used. Examples of fillers are those that themselves quickly dissolve and/or those that themselves quickly dissolve and have the ability to be brought to a pH at which drug freely dissolves in order to quickly erode the core tablet and disperse or dissolve the drug that is contained in the core tablet. Nevertheless, the preferred embodiment is not necessarily to always select filler with good solubility, that is, the preferred embodiment is to select a freely erodible filler taking into consideration physicochemical properties of the drug, particularly whether the drug is an acidic, neutral, or basic drug. Organic acids of malic acid, citric acid, and tartaric acid, particularly malic acid and citric acid, are examples of this filler when the drug is a basic drug. Sucrose, polyethylene glycol, lactulose, etc., are examples of the filler when the drug is neutral or acidic. Sucrose and polyethylene glycol are preferred.

Said filler can also be a mixture of 1 or 2 or more. It is further preferred that 1 or a mixture of 2 or more selected from malic acid, citric acid, tartaric acid, sucrose, polyethylene glycol and lactulose be used as this filler.

Malic acid and citric acid are ideal as this freely erodible filler when the 4'-[(2-methyl-1,4,5,6-tetrahydroimidazo[4,5-d] [1]benzazepin-6-yl)carbonyl]-2-phenylbenzanilide entered in International Publication No. WO 94/03305 or its pharmaceutically acceptable salt is used, or ketoconazol is used, as the drug of the present invention.

When acetaminophen is used as the drug of the present invention, polyethylene glycol is the best freely erodible filler.

Moreover, there are no special restrictions to the amount of freely erodible filler used in the present invention as long as it is an amount within the range in which the specified percentage erosion is seen, but it is approximately 10 to approximately 95%, preferably approximately 15 to approximately 80%, of the core tablet. That is, if the amount of freely erodible filler added is less than approximately 10%, a sufficient percentage erosion of the core tablet will not be obtained, while if it is greater than approximately 95%, a high percentage erosion will be seen, but there is the fear that this will cause the drug to be released in the upper digestive tract sooner than expected because overall strength of the pharmaceutical preparation will be reduced and there will therefore be cases where the goal of treatment cannot be accomplished. Moreover, there is also a chance that size of the core tablet will increase and as a result, size of the compression-coated tablet itself will increase.

It is also possible to further add to the core tablet 1 or 2 or more pharmaceutically acceptable additives for further increasing drug bioavailability so that the drug contained in the core tablet is well absorbed, even in the colon with a low water content. Examples of said additives are surfactants, such as polyoxyethylene hydrogenated castor oils, polyoxyethylene sorbitan higher fatty acid esters, polyoxyethylene polyoxypropylene glycols, sucrose fatty acid esters, etc., and the like. Moreover, the methods whereby properties of the drug itself are improved as described below are also effective. The method whereby a solid dispersion is formed between a water-soluble polymer, such as hydroxypropylmethyl cellulose, polyvinyl pyrrolidone, polyethylene glycol, etc., and an enteric polymer, such as carboxymethyl ethyl cellulose, hydroxypropyl methyl cellulose phthalate, methyl methacrylate-methacrylic acid copolymer, etc., the method of conversion to a soluble salt, the method whereby an inclusion compound is formed using cyclodextrin, etc., and the like are specific methods. Moreover, 1 or a combination of 2 or more of these methods can be used, and the above-mentioned additives and these methods can also be combined.

It is also possible to further coat the core tablet as needed. There are no special restrictions to the coating base used in the present invention as long as it is pharmaceutically acceptable and accomplishes the purpose of the present invention, and examples are polymer bases, such as hydroxypropyl methyl cellulose, etc. It is also possible to use one suitable polymer base or an appropriate combination of 2 or more polymer bases.

Hydrogel-forming polymer substance used in this compression-coated solid composition means a hydrogel-forming polymer substance that causes the compression-coated tablet of the present invention to absorb the water stagnant in the upper digestive tract and thereby gel and disintegrates after a specific amount of time as it is eroded by the contractile motion of the digestive tract that accompanies digestion of food. In particular, a substance with properties including viscosity, etc., at the time of gelling that allow the compression-coated tablet of the present invention to resist the contractile motion of the digestive tract that is the result of digestion of food in an all but completely gelled state once it has absorbed the water that is stagnant in the upper digestive tract and to migrate to the lower digestive tract as it is being eroded but still retaining its shape to a certain extent and disintegrates or is peeled at that point is an example of the hydrogel-forming polymer substance used in a preferred embodiment of the present invention. It is preferred that this polymer substance of a viscosity in an aqueous I % solution (25°C) of, for instance, 1,000 cps or higher. Moreover, the properties of the polymer substance depend on its molecular weight. Consequently, a polymer substance with a higher molecular weight is preferred to form a hydrogel capable of being used with the compression-coated tablets of the present invention. A polymer substance with a viscosity-average molecular weight of 2,000,000 or higher, further, a viscosity-average molecular weight of 4,000,000 or higher, is given as an example. Examples of said polymer substance are polyethylene oxide, such as POLYOX® WSR-303 (viscosity-average molecular weight: 7,000,000, viscosity: 7,500 to 10,000 cP (aqueous 1% solution at 25°C)), POLYOX® WSR Coagulant (viscosity-average molecular weight: 5,000,000, viscosity: 5,500 to 7,500 cP (aqueous 1% solution at 25°C)), POLYOX® WSR-301 (viscosity-average molecular weight of 4,000,000, viscosity: 1650-5500 cP (aqueous 1% solution at 25°C)), POLYOX® WSR N-60K (viscosity-average molecular weight: 2,000,000, viscosity: 2,000 to 4,000 cP (2% aqueous solution at 25°C) (all made by Union Carbide), ALKOX® E-75 (viscosity-average molecular weight: 2,000,000 to 2,500,000, viscosity: 40 to 70 cP (aqueous 0.5% solution at 25°C)), ALKOX® E-100 (viscosity-average molecular weight of 2,500,000 to 3,000,000, viscosity: 90 to 110 cP (aqueous 0.5% solution at 25°C)), ALKOX® E-130 (viscosity-average molecular weight: 3,000,000 to 3,500,000, viscosity: 130 to 140 cP (aqueous 0.5% solution at 25°C)), ALKOX® E-160 (viscosity-average molecular weight: 3,600,000 to 4,000,000, viscosity: 150 to 160 cP (aqueous 0.5% solution at 25°C)), ALKOX® E-240 (viscosity-average molecular weight: 4,000,000 to 5,000,000, viscosity: 200 to 240 cP (aqueous 0.5% solution at 25°C)) (all made by Meisei Kagaku Co., Ltd.), PEO-8 (viscosity-average molecular weight: 1,700,000 to 2,200,000, viscosity: 20 to 70 cP (aqueous 0.5% solution at 25°C)), PEO-15 (viscosity-average molecular weight: 3,300,000 to 3,800,000, viscosity: 130 to 250 cP (aqueous 0.5% solution at 25°C)), PEO-18 (viscosity-average molecular weight: 4,300,000 to 4,800,000, viscosity: 250 to 480 cP (aqueous 0.5% solution at 25°C)) (all made by Seitetsu Kagaku Kogyo Co., Ltd.), etc. However, polyethylene oxide with a molecular weight 2,000,000 or higher is particularly ideal. The polymer substance of the present invention can be 1 or a combination of 2 or more with different molecular weights, grades, etc., in order to control lag time. Moreover, a mixture with other hydrogel-forming polymer substances can also be used.

These hydrogel-forming polymer substances can also be contained in the core tablet within a range wherein the results of the timed-release pharmaceutical preparation of the present invention will not be lost. Sustained release of the drug after the lag time can be accomplished when a hydrogel-forming polymer substances is contained in the core tablet. The above-mentioned entries are examples of the hydrogel-forming polymer substance, and polyethylene oxide is preferred. The specific amount added is preferably approximately 10 to approximately 50 wt% of the core tablet.

In order for the drug to be releasable in the lower digestive tract of humans, there must be a gelled outer layer at least 2 hours after administration and the outer layer must be further disintegrated or peeled when it reaches the lower digestive tract so that the core tablet is released. Although it varies with the size of the pharmaceutical preparation, the type of polymer substance, the drug and hydrophilic base, their content, etc., the ratio of polymer substance that forms a hydrogel per total pharmaceutical preparation in order to form an outer layer with such properties in a pharmaceutical preparation of 600 mg/tablet or less is approximately 5 to approximately 95 wt% in a preferred embodiment. Approximately 10 to approximately 90 wt% is further preferred. The amount of hydrogel-forming polymer substance added per 1 tablet pharmaceutical preparation is preferably approximately 20 mg/tablet or more, particularly approximately 30 mg/tablet or more. There is a chance that the pharmaceutical preparation will not withstand contractile motion and erosion in the upper digestive tract and that the drug will therefore be released in the upper digestive tract if the amount of hydrogel-forming polymer substance is less than this amount.

Furthermore, when polyethylene oxide is used as the hydrogel-forming polymer substance, it is ideal to mix yellow ferric oxide and/or red ferric oxide as stabilizer with the outer layer of this compression-coated tablet, or to coat the compression-coated tablet with these, so that there will be no changes on release properties of the drug, even if the pharmaceutical preparation is stored exposed to light. The yellow ferric oxide or red ferric oxide used in the present invention can be used alone or as a mixture.

There are no special restrictions to the mixture ratio of yellow ferric oxide and/or red ferric oxide used in the present invention as long as it is to such an extent that the compression-coated tablets are stabilized without loosing the timed release of the present invention. This mixture ratio varies with the type and method of addition, but it is preferably approximately 1 to approximately 20 wt%, particularly approximately 3 to approximately 15 wt%, when added to the outer layer. For instance, preferably approximately 5 to approximately 20 wt%, particularly approximately 10 to approximately 15 wt%, red ferric oxide is added per the total amount of pharmaceutical preparation. Preferably approximately 1 to approximately 20 wt%, particularly approximately 3 to approximately 10 wt%, yellow ferric oxide is added. Preferably approximately 0.3 to approximately 2%, particularly approximately 0.5 to approximately 1.5 wt%, red ferric oxide or yellow ferric oxide per tablet weight is added in the case of coating with a film coating. Moreover, in this case, the concentration of yellow ferric oxide or red ferric oxide present in the film is preferably approximately 5 to approximately 50%, particularly approximately 10 to approximately 20%. When yellow ferric oxide and/or red ferric oxide are added to the outer layer, it is preferred that these iron oxides be added uniformly to the outer layer. Moreover, addition does not always mean a physical mixture. For instance, a variety of means can be adopted, such as granulation with the filler comprising the outer layer or coating granules, etc. Moreover, when the compression-coated tablets are coated, it is also possible to dissolve or suspend the above-mentioned ferric oxides in a water-soluble polymer solution such as hydroxypropyl methyl cellulose, etc., and apply a coating of a thin film using a film coating equipment, such as a High Coater (Freund Sangyo), etc. One or a combination of 2 or more of these methods can also be used.

The "hydrophilic base" contained in the compression-coated tablets of the present invention is important for the drug to reach the lower digestive tract, which has a low water content, together with water and be time-released. There are no special restrictions to the hydrophilic base as long as it can dissolve before the above-mentioned hydrogel-forming polymer substance gels. It is specifically a hydrophilic base with which the amount of water needed to dissolve 1 g of this base is 5 mL or less (20 ± 5°C), preferably 4 mL or less (same temperature). Examples of this hydrophilic base are water-soluble polymers, such as polyethylene glycol (such as Macrogol 400, Macrogol 1500, Macrogol 4000, Macrogol 6000, Macrogol 20000 (all made by Nihon Yushi)), polyvinyl pyrrolidone (for instance, PVP® K30 (made by BASF), etc., sugar alcohols, such as D-sorbitol, xylitol, etc., saccharides, such as sucrose, maltose, lactulose, D-fructose, dextran (for instance, Dextran 40), glucose, etc., surfactants, such as polyoxyethylene hydrogenated castor oil (for instance, Cremophor® RH40 (made by BASF), HCO-40, HCO-60 (made by Nikko Chemicals), polyoxyethylene polyoxypropylene glycol (for instance, Pluronic® F68 (made by Asahi Denka), etc., or polyoxyethylene sorbitan higher fatty acid esters (for instance, Tween 80 (made by Kanto Chemical), etc.), etc., salts, such as sodium chloride, magnesium chloride, etc., organic acids, such as citric acid, tartaric acid, etc., amino acids, such as glycine, β-alanine, lysine hydrochloride, etc., aminosaccharides, such as meglumine, etc., and the like. Polyethylene glycol, sucrose, and lactulose are preferred and polyethylene glycol (particularly Macrogol 6000) is further preferred. In addition, 1 or a combination of 2 or more hydrophilic bases of the present invention can be used.

When the hydrophilic base is added in the present invention, its mixture ratio is preferably approximately 5 to approximately 80 wt% in terms of the total compression-coated tablet, particularly approximately 5 to approximately 70 wt% in terms of the entire compression-coated tablet.

The hydrophilic base and freely erodible filler of the present invention can also be selected so that they duplicate on another, but as previously mentioned, the "hydrophilic base" is one with which the amount of water need to dissolve 1 g base is 5 mL or less (20 ± 5 °C), while the "freely erodible filler" is one that shows a percentage erosion of the core tablet of approximately 40 to approximately 90% when determined by the method of determining the percentage erosion. Thus, the two are differentiated based on their different functions in the present invention because they are selected in accordance with their respective definition. That is, the fact that the freely erodible filler has excellent solubility in water is one matter while the fact that the freely erodible filler has properties that will affect drugs and additives so that a constant percentage erosion of the core tablet is realized and effective timed release is obtained is yet another matter.

The mixture ratio of outer layer to the core tablet in the present invention is usually preferably approximately 0.5 to approximately 10 parts by weight, particularly approximately 1 to approximately 5 parts by weight, per 1 part by weight core tablet. Moreover, the mixture ratio of hydrophilic base and hydrogel-forming polymer substance of the outer layer is usually preferably approximately 0.1 to approximately 8 parts by weight, particularly 0.3 to approximately 5 parts by weight, hydrophilic base per 1 part by weight hydrogel-forming polymer substance.

The lag time until release of drug can be adjusted as needed taking into consideration interaction between concomitant drugs and the drug, and it can be adjusted by changing as needed the type and amount added of each component. For instance, the lag time can be adjusted based on the amount of hydrophilic base and hydrogel-forming polymer substance added to the outer layer.

By means of the present invention, other additives that are pharmaceutically acceptable can be added as needed to the core tablet and/or outer layer to such an extent that timed release of the present invention will not be lost. For instance, 1 or 2 or more of fillers, such as lactose, mannitol, potato starch, wheat starch, rice starch, corn starch, microcrystalline cellulose, etc., binders, such as hydroxypropyl methyl cellulose, hydroxypropyl cellulose, methyl cellulose, acacia, etc., swelling agents, such as carboxymethyl cellulose, carboxymethyl cellulose calcium, croscarmellose sodium, etc., lubricants, such as stearic acid, calcium stearate, magnesium stearate, talc, magnesium metasilicate, magnesium aluminate, magnesium alminosilicate, calcium hydrogen phosphate, anhydrous calcium hydrogen phosphate, etc., fluidizers, such as hydrous silicon dioxide, light anhydrous silicic acid, dry aluminum hydroxide gel, etc., coloring agents, such as yellow ferric oxide, red ferric oxide, etc., surfactants, such as sodium lauryl sulfate, sucrose fatty acid esters, etc., coating agents, such as zein, hydroxypropyl methyl cellulose, hydroxypropyl cellulose, etc., fragrances, such as 1-menthol, mentha oil, fennel oil, etc., preservatives, such as sodium sorbate, potassium sorbate, methyl parabenzoate, ethyl parabenzoate, etc., buffers, such as citric acid, succinic acid, fumaric acid, tartaric acid, ascorbic acid or salts of the same, glutamic acid, glutamine, glycine, aspartic acid, alanine, arginine or salts of the same, magnesium oxide, zinc oxide, magnesium hydroxide, phosphoric acid, boric acid or salts of the same, etc., and the like are selected in appropriate amounts.

Moreover, the oral pharmaceutical preparation of the present invention can be made by conventional production methods. The method whereby drug and freely erodible filler and when necessary, various additives, such as another filler, binder, lubricant, fluidizer, foaming agent, coloring agent, sweetener, etc., are mixed and this is made into tablets as is or after being made into granules by conventional methods and sized as necessary is given as a method of producing the core tablet. The particles can be made by conventional dry or wet granulation. For instance, after mixing the drug and each additive, the mixture can be made into granules with a screen-type granulator, a cylindrical granulator, a tornado mill, a screw granulator, or an extrusion granulator, or powder of each component can be made into granules as is using a mixing granulator. It is also possible to make granules by the fluidized bed granulation method whereby binder solution is sprayed as it component flows through the device. Next, compression molding methods referred to as press coating or dry coating, etc., are given as methods of producing a compression coated tablet. For instance, it is possible to mix the filler, binder, lubricant, fluidizer, foaming agent, coloring agent, flavoring agent, sweetener, etc., with the hydrogel-forming polymer substance and hydrophilic base of the present invention as needed, and compression coat this on a core that has already been made, or make granules by conventional methods, size these granules as needed, and then mix each additive and compression coat this on the core. The compression-coated layer can be easily prepared under ordinary conditions using an ordinary tabulating machine for making compression-coated tablets or a compression-coating device. Moreover, methods that can ordinarily be used with hydrogel preparations are mentioned as other production methods. For instance, the extrusion molding method, injection molding method, etc., can be used whereby once a core containing the drug is made, the hydrophilic base, hydrogel-forming substance and when necessary, each additive are mixed with this core, and the mixture is melted to cover the core. In addition, coating treatment such as conventional enteric coating, film coating, etc., can be performed after making tablets with a core. It is also possible to fill the tablet with a core in a capsule.

An example of the method of producing the compression-coated tablet of the present invention is shown below: A mixture of drug and freely erodible filler is granulated with an appropriate binder using a fluidized bed granulator and the granulated product is mixed with lubricant. Tablets are then made with a rotary tabulating machine to make the core tablets. The gel-forming polymer substance and hydrophilic base are separately mixed and granulated with an appropriate binder using a fluidized-bed granulator. The product is then mixed with lubricant and compression coating is performed using a tabulating machine for compression-coated tablets with this mixture serving as the outer layer of the above-mentioned core tablets to obtain the compression-coated tablets of the present invention.

The method whereby the time for which concomitant drugs and drugs coexist is made different by releasing the drug from the pharmaceutical preparation after a specific lag time (timed control) and/or the method whereby the site of absorption and metabolism or the site of impaired metabolism of the drug is made different from that of the concomitant drug by making the pharmaceutical preparation migrate to the ileum or colon where there is little CYP3A4 and being released at this site while the concomitant drugs are metabolized in the upper digestive tract, such as the duodenum, jejunum, etc., where large amounts of CYP3A4 are present (site control) are methods for alleviating pharmacological drug interaction of the present invention. A specific example is the method of using the timed-release compression-coated solid composition of the present invention.

When performing the method of alleviating drug interaction of the present invention, the above-mentioned timed-release compression-coated solid composition of the present invention is prepared by the above-mentioned method and a single dose of the pharmaceutical preparation of the present invention is administered to patients who should be administered the effective dose of a drug by timed release using this pharmaceutical preparation.

### BRIEF DESCRIPTION OF THE DRAWINGS

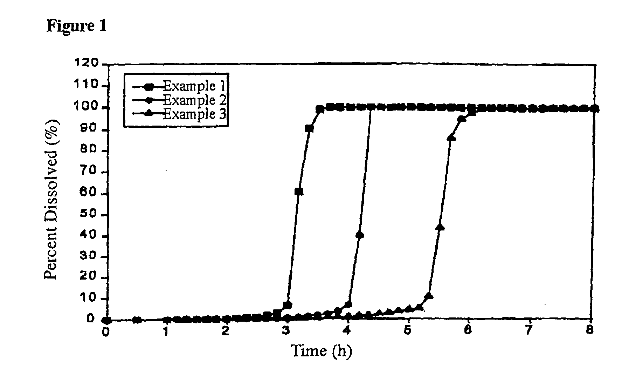

Figure 1 is a dissolution profile of Compound 1 of the pharmaceutical preparations in Examples 1 through 3.

### BEST MODE FOR CARRYING OUT THE INVENTION

The present invention is described below in further details using comparative examples, examples and test examples, but the present invention is not limited to these examples.

Incidentally, Compound 1 used in the following examples, etc., is 4'-[(2-methyl-1,4,5,6-tetrahydroimidazo[4,5-d] [1]benzazepin-6-yl)carbonyl]-2-phenylbenzanilide hydrochloride.

### Example 1

One part by weight of Compound 1, 3 parts by weight of HPMC2910, and 0.5 part by weight polysorbate 80 were dissolved in 85.5 parts by weight dichloromethane-methanol mixture (8:2) and a solid dispersion was prepared by spray drying. Then 6 parts by weight malic acid were added to 9 parts by weight solid dispersion and mixed with a mortar and pestle. A core of 150 mg per tablet with a diameter of 6.5 mm was obtained under tableting pressure of 500 kg/punch using an oil press. Separately, 50 mg polyethylene oxide (Polyox® WSR303) and 200 mg Macrogol 6000 were mixed with a mortar and pestle as the outer layer. The core was placed in the center of the outer layer and the compression-coated tablets of the present invention of 400 mg (20 mg Compound 1) per tablet with a diameter of 9.5 mm were made under a compression force of 1,000 kg/punch using an oil press.

### Example 2

One part by weight of Compound 1, 3 parts by weight of HPMC2910, and 0.5 part by weight polysorbate 80 were dissolved in 85.5 parts by weight dichloromethane-methanol mixture (8:2) and a solid dispersion was prepared by spray drying. Then 6 parts by weight malic acid were added to 9 parts by weight solid dispersion and mixed with a mortar and pestle. A core of 150 mg per tablet with a diameter of 6.5 mm was obtained under compression force of 500 kg/punch using an oil press. Separately, 62.5 mg polyethylene oxide (Polyox® WSR303) and 187.5 mg Macrogol 6000 were mixed with a mortar and pestle as the outer layer. The core was placed in the center of the outer layer and the compression-coated tablets of the present invention of 400 mg (20 mg Compound 1) per tablet with a diameter of 9.5 mm were made under a compression force of 1,000 kg/punch using an oil press.

### Example 3

One part by weight of Compound 1, 3 parts by weight of HPMC2910, and 0.5 part by weight polysorbate 80 were dissolved in 85.5 parts by weight dichloromethane-methanol mixture (8:2) and a solid dispersion was prepared by spray drying. Then 6 parts by weight malic acid were added to 9 parts by weight solid dispersion and mixed with a mortar and pestle. A core of 150 mg per tablet with a diameter of 6.5 mm was obtained under compression force of 500 kg/punch using an oil press. Separately, 87.5 mg polyethylene oxide (Polyox® WSR303) and 162.5 mg Macrogol 6000 were mixed with a mortar and pestle as the outer layer. The core was placed in the center of the outer layer and the compression-coated tablets of the present invention of 400 mg (20 mg Compound 1) per tablet with a diameter of 9.5 mm were made under a compression force of 1,000 kg/punch using an oil press.

### Example 4

One part by weight of Compound 1, 3 parts by weight of HPMC2910, and 0.5 part by weight polysorbate 80 were dissolved in 85.5 parts by weight dichloromethane-methanol mixture (8:2) and a solid dispersion was prepared by a spray drying. Then 6 parts by weight malic acid were added to 9 parts by weight solid dispersion and mixed with a mortar and pestle. A core of 150 mg per tablet with a diameter of 6.5 mm was obtained under tableting pressure of 500 kg/punch using an oil press. Separately, 71.25 mg polyethylene oxide (Polyox® WSR303), 166.25 mg Macrogol 6000 and 12.5 mg yellow ferric oxide were mixed with a mortar and pestle as the outer layer. The core was placed in the center of the outer layer and the compression-coated tablets of the present invention of 400 mg (20 mg Compound 1) per tablet with a diameter of 9.5 mm were made under a tableting pressure of 1,000 kg/punch using an oil press.

### Example 5

50 mg acetaminophen, 25 mg polyethylene oxide (Polyox® WSR303), and 75 mg sucrose were mixed with a mortar and pestle and then core tablets of 150 mg per tablet with a diameter of 6.5 mm were obtained under a compression force of 1,000 kg/punch using an oil press. 125 mg polyethylene oxide (Polyox® WSR303) and 125 mg Macrogol 6000 were separately mixed with a mortar and pestle to make the outer layer. The core tablet was placed in the center of the outer layer and compression-coated tablets of the present invention of 400 mg (50 mg acetaminophen) per tablet with a diameter of 9.5 mm were produced under a compression force of 1,000 kg/punch using an oil press.

### Example 6

50 parts by weight Macrogol 6000 were added to 100 parts by weight Diltiazem (Wako Junyaku Co., Ltd.) and mixed with a mortar and pestle. Then core tablets of 150 mg per tablet with a diameter of 7.0 mm were obtained under a compression force of 500 kg/punch using an oil press. 125 mg polyethylene oxide (Polyox® WSR303) and 175 mg Macrogol 6000 were separately mixed with a mortar and pestle to make the outer layer. The core tablet was placed in the center of the outer layer and compression-coated tablets of the present invention of 400 mg per tablet with a diameter of 10.0 mm were produced under a compression force of 1,000 kg/punch using an oil press.

### Example 7

100 parts by weight malic acid were added to 100 parts by weight ketoconazol (Sigma) and mixed with a mortar and pestle. Then core tablets of 200 mg per tablet with a diameter of 8.0 mm were obtained under a compression force of 500 kg/punch using an oil press. 150 mg polyethylene oxide (Polyox® WSR303) and 180 mg Macrogol 6000 were separately mixed with a mortar and pestle to make the outer layer. The core tablet was placed in the center of the outer layer and compression-coated tablets of the present invention of 530 mg per tablet with a diameter of 11.0 mm were produced under a compression force of 1,000 kg/punch using an oil press.

### Example 8

1 part by weight Compound 1, 3 parts by weight HPMC 2910, and 0.5 parts by weight polysorbate 80 were dissolved in 85.5 parts by weight dichloromethane-methanol mixture (8:2) and a solid dispersion was prepared by spray drying. Then 8 parts by weight malic acid were added to 9 parts by weight of the solid dispersion and mixed with a mortar and pestle. Core tablets of 170 mg per tablet with a diameter of 8 mm were obtained under a compression force of 500 kg/punch using an oil press. 150 mg polyethylene oxide (Polyox® WSR303) and 180 mg Macrogol 6000 were separately mixed with a mortar and pestle to make the outer layer. The core tablet was placed in the center of the outer layer and compression-coated tablets of the present invention of 500 mg (20 mg Compound 1) per tablet with a diameter of 11 mm were produced under a compression force of 1,000 kg/punch using an oil press.

### Example 9

1 part by weight Compound 1, 3 parts by weight HPMC 2910, and 0.5 parts by weight polysorbate 80 were dissolved in 85.5 parts by weight dichloromethane-methanol mixture (8:2) and a solid dispersion was prepared by spray drying. Then 6 parts by weight malic acid were added to 9 parts by weight of the solid dispersion and mixed with a mortar and pestle. Core tablets of 150 mg per tablet with a diameter of 6.5 mm were obtained under a compression force of 500 kg/punch using an oil press. 75 mg polyethylene oxide (Polyox® WSR303) and 225 mg Macrogol 6000 were separately mixed with a mortar and pestle to make the outer layer. The core tablet was placed in the center of the outer layer and compression-coated tablets of the present invention of 450 mg (20 mg Compound 1) per tablet with a diameter of 9.5 mm were produced under a compression force of 1,000 kg/punch using an oil press.

### Test Example 1

### Comparative example 1

1 part by weight Compound 1, 3 parts by weight HPMC 2910, and 0.5 parts by weight polysorbate 80 were dissolved in 85.5 parts by weight dichloromethane-methanol mixture (8:2) and a solid dispersion was prepared by spray drying. Then 6 parts by weight lactose were added to 9 parts by weight of the solid dispersion and mixed with a mortar and pestle. Core tablets of 150 mg per tablet with a diameter of 6.5 mm were obtained under a tabulating pressure of 500 kg/punch using an oil press. 75 mg polyethylene oxide (Polyox® WSR303) and 225 mg Macrogol 6000 were separately mixed with a mortar and pestle to make the outer layer. The core tablet was placed in the center of the outer layer and compression-coated tablets of the present invention of 450 mg (20 mg Compound 1) per tablet with a diameter of 9.5 mm were produced under a tabulating pressure of 1,000 kg/punch using an oil press.

### (Determination of percentage erosion of core tablet)

The percentage erosion of the core tablets in example 9 and comparative example 1 was determined in accordance with the above-mentioned method of determining the percentage erosion of core tablets.

### (Administration to dog)

The above-mentioned pharmaceutical preparations (20 mg Compound 1/dog) were orally administered with 30 mL water to male beagles (n = 5) that had been fasted for approximately 20 hours. After administration, blood was collected over time from the veins of the front limbs and concentration of Compound 1 in plasma was determined by the HPLC/UV method. The area under plasma concentration curve (AUC) was calculated.

### (Results and discussion)

The percentage erosion of the core tablet of example 9, wherein the core tablet contained malic acid, was 70%, while that of comparative example 1, wherein the core tablet contained lactose, was 24%. As a result of performing experiments by administration to dogs, the drug concentration in blood could be detected after a lag time of approximately 3 hours with both pharmaceutical preparations. The AUC was lower with administration of comparative example 1, which had a low percentage erosion of core tablet, than with administration of example 9, which had a high percentage erosion. Consequently, this indicates that a high percentage erosion of the core tablet when drug is released after timed release, particularly in the lower digestive tract, is important for maintaining bioavailability.

**Table 1.**

| Mean AUC when example 9 and comparative example 1 were administered | |
|---|---|
| | AUC (ng·h/ml) |
| Example 9 | 5299 |
| Comparative example 1 | 3969 |

### Test Example 2

### Comparative example 2

50 mg acetaminophen, 25 mg polyethylene oxide, and 75 mg lactose were mixed with a mortar and pestle and then core tablets of 150 mg per tablet with a diameter of 6.5 mm were obtained under a tabulating pressure of 1,000 kg/punch using an oil press. 125 mg polyethylene oxide (Polyox® WSR303) and 125 mg Macrogol 6000 were separately mixed with a mortar and pestle to make the outer layer. The core tablet was placed in the center of the outer layer and compression-coated tablets of the present invention of 400 mg (50 mg acetaminophen) per tablet with a diameter of 9.5 mm were produced under a tabulating pressure of 1,000 kg/punch using an oil press.

### (Administration to dog 1)

.The pharmaceutical preparation of example 5 was orally administered (50 mg/dog) with 30 mL water to male beagles (n = 6) that had been fasted for approximately 20 hours. After administration, blood was collected over time from the veins of the front limbs and the acetaminophen concentration in plasma was determined by the HPLC/UV method. The area under plasma concentration curve (AUC) and the first appearance time of drug in plasma (FAT) were calculated.

### (Administration to dog 2)

The pharmaceutical preparation of comparative example 2 (50 mg/dog) was orally administered with 30 mL water to male beagles (n =6) that had been fasted for approximately 20 hours. After administration, blood was collected over time from the veins of the front limbs and the acetaminophen concentration in plasma was determined by the HPLC/UV method. The area under plasma concentration curve (AUC) and first appearance time of drug in plasma (FAT) were calculated.

### (Determination of percentage erosion of core tablet)

The percentage erosion of the core tablet of both of the above-mentioned pharmaceutical preparations was determined in accordance with the previously described method of determining the percentage erosion of the core tablet.

### (Results and discussion)

As a result of evaluating both of the above-mentioned pharmaceutical preparations by core tablet erosion tests, that of the pharmaceutical preparation of example 5 was 55.2% and that of the pharmaceutical preparation of comparative example 2 was 24.8%.

The bioavailability of the two pharmaceutical preparations with different percentages erosion of the core tablet was compared (Table 2). The same results were seen in terms of the first appearance time of drug in plasma (lag time), but the AUC of the pharmaceutical preparation of the comparative example 2 with a low percentage erosion was low in comparison to the pharmaceutical preparation of example 5. This therefore indicates that a high percentage erosion of the core tablet after timed release, particularly when a drug is released in the lower digestive tract, is important in maintaining bioavailability.

**(Table 2)**

| Mean AUC and first appearance time when pharmaceutical preparations administered | | |
|---|---|---|
| | AUC (ng·h/ml) | FAT (h) |
| Example 5 | 1054 | 4.0 |
| Comparative example 2 | 387 | 4.0 |
| FAT: first appearance time of drug in plasma | | |

### Test example 3

The following test was performed in order to clarify the correlation between solubility of each filler and the percentage erosion of the compression-coated tablet containing this filler: One part by weight of Compound 1, 3 parts by weight of HPMC2910, and 0.5 part by weight polysorbate 80 were dissolved in 85.5 parts by weight dichloromethane-methanol mixture (8:2) and a solid dispersion was prepared by spray drying. Then 6 parts by weight of each filler were added to 9 parts by weight of the solid dispersion and mixed with a mortar and pestle. A core tablet of 150 mg per tablet with a diameter of 6.5 mm was obtained under compression force of 500 kg/punch using an oil press. Moreover, 10 parts by weight of each filler were separately added to 5 parts by weight acetaminophen (Yoshitomi Seiyaku) and mixed with a mortar and pestle and core tablets of 150 mg per tablet with a diameter of 6.5 mm were obtained under compression force of 500 kg/punch using an oil press. Furthermore, 75 mg polyethylene oxide (Polyox® WSR303) and 175 mg Macrogol 6000 were mixed with a mortar and pestle as the outer layer. The above-mentioned core tablets were placed in the center (core tablets containing Compound 1 were placed in the center to observe the effect of each filler on Compound 1 and core tablets containing acetaminophen were placed in the center to observe the effect of each filler on acetaminophen) and compression-coated tablets of the present invention of 400 mg per tablet (20 mg Compound 1, 50 mg acetaminophen) with a diameter of 9.5 mm were produced under a compression force of 1,000 kg/punch using an oil press. This tablet was immersed for 3 hours in water at 37°C and then the gelated part of the tablet was peeled off and the uneroded core was removed. The core was dried in a dryer overnight at 40°C and then weighed. The percentage erosion of the core tablet was calculated from the dry weight and initial weight.

### (Results and discussion)

The results of the compression-coated tablet containing Compound 1 are shown in Table 3 and the results of the compression-coated tablet containing acetaminophen are shown in Table 4. A high percentage erosion was obtained with citric acid, tartaric acid and malic acid when Compound 1 was used, while a good percentage erosion was obtained with malic acid and polyethylene glycol 6000 when acetaminophen was used. Moreover, it was shown that the percentage erosion is not necessarily good with a filler having good solubility and it is therefore necessary to select the appropriate filler in accordance with the drug contained in the tablet.

**(Table 3)**

| Solubility of each filler and percentage erosion of compression-coated tablet containing Compound 1 (%) | | |
|---|---|---|
| Filler | Solubility | Percentage erosion (%) |
| Citric acid | 1 mL | 60.3 |
| Tartaric acid | 1mL | 65.7 |
| Malic acid | 2 mL | 75.0 |
| PEG6000 | 1 mL | 42.6 |
| Sucrose | 1 mL | 48.4 |
| PVPK30 | 2 mL | 47.6 |
| Ascorbic acid | 6 mL | 32.4 |
| Succinic acid | 10 mL< | 19.2 |
| Fumaric acid | 10 mL< | 2.0 |
| Aspartic acid | 10 mL< | 1.4 |
| Lactose | 8 mL | 24.0 |
| ^{*}Solubility is represented by the amount of water needed to dissolve 1 g filler. | | |

**(Table 4)**

| Solubility of each filler and percentage erosion (%) of compression-coated tablets containing acetaminophen | | |
|---|---|---|
| Filler | Solubility | Percentage erosion (%) |
| Malic acid | 2 mL | 79.3 |
| PEG6000 | 1 mL | 84.1 |
| Sucrose | 1 mL | 56.1 |
| Succinic acid | 10 mL< | 12.8 |
| Lactose | 8 mL | 7.6 |
| ^{*} Solubility was represented by the amount of water needed to dissolve 1 g filler. | | |

### Test example 4

Dissolution tests were performed on the preparations in Examples 1 through 3. The tests were conducted by the Second Dissolution Testing Method (Paddle Method) of the Pharmacopoeia of Japan (paddle rotation: 200 rpm) using 500 ml of 1st fluid of the Disintegration Testing Method of the Pharmacopoeia of Japan as the dissolution medium. Sampling was performed each hour and the amount of Compound 1 in the sampled solution was determined by the UV method.

### (Results)

The results of the Dissolution tests are shown in Figure 1. The figure confirms that Compound 1 is first released from the preparation of the present invention after a specific amount of time. Moreover, the results show that the lag time until release starts can be adjusted by adjusting the mixture ratio of the polyethylene glycol and polyethylene oxide of the outer layer.

### Test example 5

The following experiments were conducted using midazolam, which is metabolized by CYP3A4, as a concomitant drug of Compound 1:

### (Preparation of Sample Solution)

(1) Aqueous solution for oral administration containing midazolam: After preparing commercial midazolam injectable liquid (brand name: Dormicum® injection, made by Roche Co., Ltd., marketed by Yamanouchi Pharmaceutical) to a concentration of 0.2 mg/ml with aqueous hydrochloric acid solution (pH of 3), HPMC2910 was added at 3-times the amount of midazolam to obtain a liquid for oral administration.
(2) Aqueous solution for oral administration containing Compound 1: Compound 1 was dissolved to a concentration of 0.5 mg/ml with an aqueous hydrochloric acid solution (pH of 3) to obtain a liquid for oral administration.

### (Experiment 1)

Male beagle dogs (n = 6) that had been fasted for approximately 20 hours were orally administered the aqueous solution for oral administration containing midazolam using a catheter for oral administration (4 mg/dog). After administration, blood was collected from the veins of the front legs and the plasma concentration of midazolam was determined by the HPLC/UV method over time.

### (Experiment 2)

Male beagle dogs (n = 6) that had been fasted for approximately 20 hours were orally administered the aqueous solution for oral administration containing Compound 1 using a catheter for oral administration (10 mg/dog). Thirty minutes after administration the aqueous solution for oral administration containing midazolam was orally administered using a catheter for oral administration (4 mg/dog). After midazolam administration, blood was collected from the veins of the front legs and the plasma concentration of midazolam was determined by the HPLC/UV method over time.

### (Experiment 3)

Male beagle dogs (n = 6) that had been fasted for approximately 20 hours were orally administered the preparation of Example 2 (20 mg/dog) with 30 ml of water. Thirty minutes after administration an aqueous solution for oral administration containing midazolam (4 mg/dog) was orally administered using a catheter for oral administration. After midazolam administration, blood was collected from the veins of the front legs and the plasma concentration of midazolam was determined by the HPLC/UV method over time.

### (Results and Discussion)

As is clear from the results of Experiments 1 and 2, when the aqueous solution for oral administration containing Compound 1 was concomitantly used by oral administration before oral administration of the midazolam, significant changes were seen in that there was significant elevation of the midazolam blood concentration and the area under plasma concentration curve (AUC) increased by at least 2-fold, etc., when compared to singular oral administration of midazolam (Table 5). The reason for this apparently is that Compound 1, which has the same route of metabolism by CYP3A4 inhibits metabolism of midazolam in the small intestine and as a result, there is a an increase in the midazolam blood concentration and AUC.

**Table 5.**

| Mean AUC of midazolam plasma concentration | |
|---|---|
| | AUC (ngh/ml) |
| Experiment 1 (Midazolam singular administration) | 9.0 ± 6.0 |
| Experiment 2 (Concomitant use of aqueous solution for oral administration containing Compound 1) | 21.2 ±8.5^{*} |
| Experiment 3 (Concomitant use of preparation of Example 2) | 10.9 ± 7.3 |

| | |
|---|---|
| ^{*}p < 0.05 (to Experiment 1) | |

On the other hand, as is clear from the results of Experiment 1 and Experiment 3 when the preparation of Example 2 was concomitantly used by oral administration before oral administration of the midazolam, the midazolam blood concentration and AUC showed almost the same result as with midazolam singular administration (Table 5). From this finding it appears that by means of the preparation of the present invention, metabolism of the midazolam by CYP3A4 in the small intestine is not inhibited by Compound 1 because Compound 1 is released after the midazolam has been metabolized by CYP3A4 in the upper small intestine and as a result, there is no effect on the midazolam blood concentration or AUC. Moreover, it was confirmed that the blood concentration of Compound 1 was enough to provide pharmacologically the therapeutic or prophylactic effect of Compound 1 once the midazolam had cleared from the blood.

### Test example 6

The following experiments were performed using diltiazem, which inhibits metabolism by CYP3A4, and midazolam, which is metabolized by CYP3A4:

### (Preparation of sample solutions)

(1) Aqueous solution for oral administration containing midazolam: After preparing a commercial midazolam injectable solution (brand name: Dormicum® Injectable; manufactured by Roche, marketed by Yamanouchi Pharmaceutical) with aqueous hydrochloric acid solution (pH 3) so that the concentration was 0.2 mg/mL, HPMC 2910 was added at 3-times the amount of midazolam to obtain the solution for oral administration.
(2) Aqueous solution for oral administration containing diltiazem: Diltiazem was dissolved to a concentration of 20 mg/mL to obtain the solution for oral administration.

### (Experiment 4)

The aqueous solution for oral administration containing midazolam was orally administered (4 mg/dog) using a catheter for oral administration to male beagle dogs (n = 3) that had been fasted for approximately 20 hours. After administration, blood was collected from the veins of the front limbs of the dogs and the midazolam concentration of the plasma was determined by the HPLC/UV method.

### (Experiment 5)

The aqueous solution for oral administration containing diltiazem was orally administered (200 mg/dog) using a catheter for oral administration, while at the same time, the aqueous solution for oral administration of midazolam was orally administered (4 mg/dog) using a catheter for oral administration to male beagle dogs (n = 3) that had been fasted for approximately 20 hours. After administration, blood was collected from the veins of the front limbs of the dogs and the midazolam concentration of the plasma was determined by the HPLC/UV method.

### (Experiment 6)

The pharmaceutical preparation of example 6 was orally administered (200 mg/dog) with 30 ml water using a catheter for oral administration, while at the same time, the aqueous solution for oral administration of midazolam was orally administered (4 mg/dog) using a catheter for oral administration to male beagle dogs (n = 3) that had been fasted for approximately 20 hours. After administration, blood was collected from the veins of the front limbs of the dogs and the midazolam concentration of the plasma was determined by the HPLC/UV method.

### (Results and discussion)

As is clear from the results of Experiment 4 and Experiment 5, when an aqueous solution for oral administration containing diltiazem was concomitantly used by oral administration simultaneously with midazolam oral administration, there was a marked rise in the blood concentration of midazolam and the average area under plasma concentration curve (AUC) increased by as much as 3-times (Table 6) when compared to midazolam singular administration. The reason for this is apparently that diltiazem inhibits metabolism by CYP3A4 and as a result, induces an increase in the midazolam AUC.

**(Table 6)**

| Mean AUC of midazolam plasma concentration | |
|---|---|
| | AUC (ng·h/ml) |
| Experiment 4 (midazolam singular administration) | 55.0 |
| Experiment 5 (concomitant use of aqueous solution for oral administration containing diltiazem) | 163.0 |
| Experiment 6 (concomitant use of pharmaceutical preparation of Example 6) | 48.9 |

On the other hand, as is clear from the results in Experiment 4 and Experiment 6 almost the same results as with midazolam singular administration are seen in terms of the midazolam AUC when the pharmaceutical preparation of Example 6 was concomitantly used by oral administration simultaneously with midazolam oral administration (Table 6). Thus, it appears that when the pharmaceutical preparation of the present invention is used, diltiazem is released after midazolam has been metabolized by CYP3A4 in the upper small intestine and therefore had no effect on the AUC of midazolam.

### Test example 7

The following experiments were conducted using ketoconazol, which inhibits metabolism by CYP3A4 and midazolam, which is metabolized by CYP3A4.

### (Preparation of sample solutions)

(1) Aqueous solution for oral administration containing midazolam: After preparing a commercial midazolam injectable solution (brand name: Dormicum® Injectable; manufactured by Roche, marketed by Yamanouchi Pharmaceutical) with aqueous hydrochloric acid solution (pH 3) so that the concentration was 0.2 mg/mL, HPMC 2910 was added at 3-times the amount of midazolam to obtain the solution for oral administration.
(2) Aqueous solution for oral administration containing ketoconazol: Ketoconazol was dissolved to a concentration of 5 mg/mL to obtain the solution for oral administration.

### (Experiment 7)

The aqueous solution for oral administration containing midazolam was orally administered (4 mg/dog) using a catheter for oral administration to male beagle dogs (n = 2) that had been fasted for approximately 20 hours. After administration, blood was collected from the veins of the front limbs of the dogs and the midazolam concentration of the plasma was determined by the HPLC/UV method.

### (Experiment 8)

The aqueous solution for oral administration containing ketoconazol was orally administered (100 mg/dog) using a catheter for oral administration, while at the same time, the aqueous solution for oral administration of midazolam was orally administered (4 mg/dog) using a catheter for oral administration to male beagle dogs (n = 2) that had been fasted for approximately 20 hours. After administration, blood was collected from the veins of the front limbs of the dogs and the midazolam concentration of the plasma was determined by the HPLC/UV method.

### (Experiment 9)

The pharmaceutical preparation of example 7 was orally administered (200 mg/dog) with 30 ml water, while at the same time, the aqueous solution for oral administration of midazolam was orally administered (4 mg/dog) using a catheter for oral administration to male beagle dogs (n = 2) that had been fasted for approximately 20 hours. After administration, blood was collected from the veins of the front limbs of the dogs and the midazolam concentration of the plasma was determined by the HPLC/UV method.

### (Results and discussion)

As is clear from the results of Experiment 7 and Experiment 8, when an aqueous solution for oral administration containing ketoconazol was concomitantly used by oral administration before midazolam oral administration, there was a marked rise in the blood concentration of midazolam and the average area under plasma concentration curve (AUC) increased by as much as 4-times (Table 7) when compared to midazolam singular administration. The reason for this is apparently that ketoconazol inhibits metabolism by CYP3A4 and as a result, induces an increase in the midazolam AUC.

**(Table 7)**

| Mean AUC of midazolam plasma concentration | |
|---|---|
| | AUC (ng·h/ml) |
| Experiment 7 (midazolam singular administration) | 68.7 |
| Experiment 8 (concomitant use of aqueous solution for oral administration containing ketoconazol) | 245.5 |
| Experiment 9 (concomitant use of pharmaceutical preparation of Example 7) | 69.5 |

On the other hand, as is clear from the results of Experiment 7 and Experiment 9, when the pharmaceutical preparation of Example 7 was concomitantly used by oral administration simultaneously with midazolam oral concentration, the midazolam AUC showed approximately the same result as with midazolam singular administration (Table 7). Thus, it appears that when the pharmaceutical preparation of the present invention is used, ketoconazol is released after midazolam has been metabolized by CYP3A4 in the upper small intestine and therefore had no effect on the AUC of midazolam.

### Test example 8

The following experiments were conducted using Compound 1, which inhibits metabolism by CYP3A4 and simvastatin, which is metabolized by CYP3A4:

### (Preparation of sample solutions)

(1) Aqueous solution for oral administration containing Compound 1: PEG 200, 5 mM phosphoric acid, and Compound 1 were mixed at a ratio of 1:1:8 and prepared so that the concentration would be 1.67 mg/mL to obtain the solution for oral administration.
(2) Simvastatin pharmaceutical preparation for oral administration: Commercial Lipovas tablets (Banyu Seiyaku) were used.

### (Experiment 10)

Simvastatin pharmaceutical preparation for oral administration was orally administered (25 mg/monkey) to male cynomologous monkeys (n = 6) that had been fasted for approximately 12 hours on Test Day 1. Blood was collected from the femoral vein over time following administration and the simvastatin concentration of plasma was determined by the LC/MS/MS method. An aqueous solution for oral administration containing Compound 1 was orally administered (5 mg/kg) using a nasogastric tube to male cynomologous monkeys (n= 6) that had been fasted for approximately 12 hours or longer each morning from Test Day 2 up to Test Day 6. Simvastatin pharmaceutical preparation for oral administration was orally administered (25 mg/monkey) simultaneously with oral administration of the aqueous solution for oral administration containing Compound 1 on Test Day 6. Blood was collected from the femoral vein over time following administration and the simvastatin concentration of plasma was determined by the LC/MC/MS method.

### (Experiment 11)

Simvastatin pharmaceutical preparation for oral administration was orally administered (25 mg/monkey) to male cynomologous monkeys (n = 6) that had been fasted for approximately 12 hours or longer on Test Day 1. Blood was collected from the femoral vein over time once administration was completed and the simvastatin concentration of plasma was determined by the LC/MS/MS method. The pharmaceutical preparation of example 8 was orally administered (20 mg/monkey) to male cynomologous monkeys (n = 6) that had been fasted for approximately 12 hours or longer each morning from Test Day 2 up to Test Day 6. On Test Day 6, simvastatin pharmaceutical preparation for oral administration was orally administered (25 mg/monkey) simultaneously with oral administration of the pharmaceutical preparation of example 8. Blood was collected from the femoral vein over time once administration was completed and the simvastatin concentration of plasma was determined by the LC/MS/MS method.

### (Results and discussion)

As is clear from the results of Experiment 10 and Experiment 11, there were marked changes when simvastatin pharmaceutical preparation for oral administration was concomitantly used by oral administration simultaneously after continuous administration of the aqueous solution for oral administration of Compound 1 in that the simvastatin concentration in blood rose markedly and the AUC was approximately 24-times or more that of singular administration, etc., when compared to simvastatin singular oral administration (Table 8). The reason for this is apparently that Compound 1 inhibits metabolism by CYP3A4 and as a result, induces an increase in the simvastatin AUC.

On the other hand, as is clear from the results of Experiment 10 and Experiment 11, when the pharmaceutical preparation of Example 8 was concomitantly used by oral administration simultaneously with simvastatin pharmaceutical preparation for oral administration after being continuously administered, the simvastatin AUC was 4-times higher when compared to simvastatin singular administration and was very low when compared to concomitant use with the aqueous solution for oral administration of Compound 1 (Table 8). Thus, it appears that when the pharmaceutical preparation of the present invention is used, compound 1 is released after simvastatin has been metabolized by CYP3A4 in the upper small intestine and therefore, a rise in the simvastatin AUC can be controlled.

**(Table 8)**

| Mean AUC of simvastatin concentration in plasma | | | |
|---|---|---|---|
| | | AUC (ng·h/mL) | AUC ratio (Test Day 6/Test Day 1) |
| Experiment | | Average | Average (range) |
| 10 | Simvastatin singular administration (Test Day 1) | 62.7 | -- |
| | Aqueous Compound 1 solution concomitant administration (Test Day 6) | 806.5 | x 24.1 (54.1-5.8) |
| 11 | Simvastatin singular administration (Test Day 1) | 65.2 | -- |
| | Aqueous Example 8 solution concomitant administration (Test Day 6) | 324.3 | x 4.1(7.4-0.2) |

### INDUSTRIAL APPLICABILITY

The timed-release compression-coated solid composition for oral administration of the present invention has advantages in that erosion of the core tablet is good and there is no reduction in bioavailability as a timed-released pharmaceutical preparation. Consequently, it is useful as presented as a timed-release pharmaceutical preparation that is ideal for various conditions in which timed-release of a drug is appropriate and their treatment protocol.

That is, the timed-release compression-coated solid composition for oral use of the present invention makes it possible for the drug to specifically reach the afflicted site in the lower digestive tract by releasing the drug after a specific lag time. Moreover, the composition of the present invention makes effective absorption in the lower digestive tract possible. Furthermore, the present invention makes it possible to realize efficacy only at the time period that is significant in terms of chronopharmacotherapy. The present invention further makes it possible to alleviate pharmacokinetic drug interaction between a drug and concomitant drugs by making the time for which concomitant drugs and the drug coexist separate (time control) and/or by allowing the pharmaceutical preparation to migrate to the ileum or colon where there is little CYP3A4 so that drug is released at that site and thereby making the site of absorption or metabolism different from that of the concomitant drugs (site control by time control) and thus making it possible to alleviate the effect of competition over metabolism or inhibit metabolism in the epithelial cells of the small intestine, or by making the time for which the drug that has been absorbed *in vivo* (intravascularly) coexists with concomitant drug in the liver different. As a result, drug is released in the ileum or colon where there is little CYP3A4 and pharmacokinetic drug interaction relating to CYP3A4 in the digestive tract can be averted and *in vivo* absorption becomes possible. Pharmacokinetic changes of concomitant drugs can be controlled by administration of the compression-coated solid composition of the present invention.

The timed-release compression-coated solid composition for oral administration of the present invention in particular makes timed release possible, even in the lower digestive tract where release has been difficult in the past, as a result of A. absorption of water stagnant in the upper digestive tract so that the outer layer all but completely gels, B. penetration of the water to inside the core tablet so that the core tablet freely erodes to become a solution state or suspension state, C. erosion of the gelled outer layer as it moves to the lower digestive tract, and D. further disintegration or peeling of part of the outer layer to release drug.

## Claims

1. A timed-release compression-coated solid composition for oral administration, said composition being a hydrgel-forming compression-coated solid preparation comprising a core tablet comprising a drug and an outer layer comprising a hydrogel-forming polymer substance and a hydrophilic base, **characterized in that**:
(1) said core tablet comprising a drug and a freely erodible filler,
(2) said core tablet is capable of approximately 40 to approximately 90% erosion; and
(3) said outer layer substantially does not contain the same drug as said drug.

2. The timed-release compression-coated solid composition for oral administration according to claim 1, which comprises approximately 75 wt% or less of said drug, approximately 5 to approximately 80 wt% freely erodible filler, approximately 10 to approximately 95 wt% hydrogel-forming polymer substance, and approximately 5 to approximately 80 wt% hydrophilic base.

3. The timed-release compression-coated solid composition for oral administration according to claim 1, wherein the freely erodible filler is 1 or 2 or more selected from the group consisting of malic acid, citric acid, tartaric acid, polyethylene glycol, sucrose, and lactulose.

4. The timed-release compression-coated solid composition for oral administration according to claim 1, wherein the freely erodible filler is 1 or 2 or more selected from the group consisting of malic acid, citric acid and tartaric acid.

5. The timed-release compression-coated solid composition for oral administration according to claim 1, wherein the freely erodible filler for a basic drug is 1 or 2 or more selected from the group consisting of malic acid, citric acid and tartaric acid.

6. The timed-release compression-coated solid composition for oral administration according to claim 1, wherein the freely erodible filler for an acidic or neutral drug is 1 or 2 or more selected from the group consisting of polyethylene glycol, sucrose or lactulose.

7. The timed-release compression-coated solid composition for oral administration according to claim 1, wherein the hydrogel-forming polymer substance contains at least one type of polyethylene oxide.

8. The timed-release compression-coated solid composition for oral administration according to claim 1, wherein the hydrogel-forming polymer substance is 1 or 2 or more having a viscosity-average molecular weight of 2,000,000 or higher and/or a viscosity in an aqueous 1% solution (25°C) of 1,000 cp or higher.

9. The timed-release compression-coated solid composition for oral administration according to claim 1, wherein the core tablet comprises a hydrogel-forming polymer substance.

10. The timed-release compression-coated solid composition for oral administration according to claim 1, wherein the hydrophilic base is 1 or 2 or more having solubility such that the amount of water needed to dissolve 1 g base is 5 mL or less.

11. The timed-release compression-coated solid composition for oral administration according to claim 10, wherein the hydrophilic base is 1 or 2 or more selected from the group consisting of polyethylene glycol, sucrose, and lactulose.

12. The timed-release compression-coated solid composition for oral administration according to claim 1, wherein the hydrogel-forming polymer substance is at least 1 type of polyethylene oxide and further comprises red ferric oxide and/or yellow ferric oxide.

13. The timed-release compression-coated solid composition for oral administration according to claim 1, wherein the drug is brought to be effectively released or absorbed in the lower digestive tract.

14. The timed-release compression-coated solid composition for oral administration according to claim 1, wherein the drug is brought to be effective for chronopharmacotherapy.

15. The timed-release compression-coated solid composition for oral administration according to any one of claims 1 to 14, wherein the drug is metabolized by cytochrome P-450.

16. The timed-release compression-coated solid composition for oral administration according to any one of claims 1 to 14, wherein the drug has the effect of inhibiting metabolism by cytochrome P-450.

17. The timed-release compression-coated solid composition for oral administration according to claim 15, wherein the drug is metabolized by CYP3A4.

18. The timed-release compression-coated solid composition for oral administration according to claim 16, wherein the drug has the effect of inhibiting metabolism by CYP3A4.

19. The timed-release compression-coated solid composition for oral administration according to claim 1, wherein the drug is 4'-[(2-methyl-1,4,5,6-tetrahydroimidazo[4,5-d][1]benzazepin-6-yl)carbonyl]-2-phenylbenzanilide or a salt thereof.

20. A method of timed release of a drug, which comprises orally administering the composition described in any one of claims 1 to 19.

21. A method of alleviating undesirable drug interaction between a drug and another drug used concomitantly that employ the same route for drug absorption, distribution, metabolism or excretion *in vivo* in humans, which comprises using the composition described in any one of claims 1 to 19.

22. A method of alleviating undesirable drug interaction between a drug having the effect of inhibiting drug metabolism *in vivo* in humans and another drug according to claim 19 used concomitantly, which comprises using the composition described in any one of claims 1 to 19.

23. Use of a timed-release compression-coated solid composition for oral administration described in any one of claims 1 to 19, for alleviating undesirable drug interaction between a drug and another drug used concomitantly that employ the same route for drug absorption, distribution, metabolism or excretion *in vivo* in humans.

24. Use of a timed-release compression-coated solid composition for oral administration described in any one of claims 1 to 19, for alleviating undesirable drug interaction between a drug having the effect of inhibiting drug metabolism *in vivo* in humans and another drug according to claim 23 used concomitantly.

25. Use of a drug which is brought to be effective for chronopharmacotherapy, for manufacturing a timed-release compression-coated solid composition for oral administration described in any one of claims 1 to 19, for alleviating undesirable drug interaction between a drug and another drug used concomitantly that employ the same route for drug absorption, distribution, metabolism or excretion *in vivo* in humans.

26. Use as described in claim 25, for manufacturing a timed-release compression-coated solid composition for oral administration described in any one of claims 1 to 19, for alleviating undesirable drug interaction between a drug having the effect of inhibiting drug metabolism *in vivo* in humans and another drug according to claim 23 used concomitantly.
